# NEW EUROPEAN PATENT SPECIFICATION

(11) **EP 1 845 933 B2**
(45) Date of publication and mention of the opposition decision: **22.06.2016**
(45) Mention of the grant of the patent: 30.10.2013
(21) Application number: 06700445.7
(22) Date of filing: 12.01.2006
(51) Int. Cl.: A61K 8/29, A61K 8/02, A61K 8/25, A61Q 11/00

(54) **MULTIPHASE TOOTHPASTE**
MEHRPHASIGE ZAHNPASTENZUSAMMENSETZUNG
COMPOSITION DE DENTIFRICE MULTIPHASE

(30) Priority: 04.02.2005 IN MU01182005; 08.04.2005 IN KO02932005
(43) Date of publication of application: 24.10.2007
(73) Proprietor: Unilever N.V., 3013 AL Rotterdam (NL); Unilever PLC, London EC4Y 0DY (GB)
(72) Inventor: AGARWAL, Rupesh Kumar, Mumbai 400 099 (IN); FARAVELLI, Ilaria, I-26841 Casalpusterlengo (IT); GREGORY, Donald Peter, Bebington, Wirral Merseyside, CH63 3JW (GB); GROVES, Brian, Bebington, Wirral Merseyside CH63 3JW (GB); ROBERTS, Geraint Paul, Bebington, Wirral Merseyside CH63 3JW (GB)
(74) Representative: Potter Clarkson LLP
(86) International application number: PCT/EP2006/000236
(87) International publication number: WO 2006/081924

(56) References cited:
- GB-A- 1 271 944

## Description

The present invention relates to toothpaste comprising a first composition and a second composition coaxially arranged within the first composition. Each composition comprises a humectant, a thickener and water.

WO 99/01342 discloses apparatus for inserting plural materials into containers. The apparatus comprises a nozzle with a first hollow member and a second hollow member arranged inside the first hollow member. The nozzle is designed for directing the extrusion of multiple toothpaste phases into a toothpaste container with one phase being arranged within another.

The invention provides an improvement for toothpastes comprising one phase disposed coaxially within another.

The second phase is disposed co-axially within the first phase. By this is meant that the longitudinal axis of the ribbon as dispensed from the toothpaste container falls within the inner, second phase. Similarly, the general longitudinal axis of the toothpaste as stored within the toothpaste container falls within the inner, second phase. Such alignment is understood to be judged by the eye and not mathematically.

The inner phase comprises no extensions and instead provides nothing more than a regular core to the ribbon. This regular shape is less fussy and provide clean lines to the product which reinforces the impression of cleanliness that toothpastes aim to provide.

Accordingly, the present invention provides a toothpaste which comprises first and second compositions, the first and second compositions comprising water, humectant and thickener and the second composition disposed coaxially within the first composition, the first composition being a gel and the second composition being opaque, the second composition comprising an opacifier at y% by weight of the second composition and the first composition being substantially devoid of any opacifier comprising 0.2 to 2.0y% by weight of the first composition more thickener than is present as a weight percent of the second composition.

The careful balancing of thickening materials allows the toothpaste of the present invention to be optimally extruded into a toothpaste tube using a nozzle as disclosed in WO 99/01342. This balancing needs to be carefully managed because of the change in rheology brought about by the opacifier.

The opacifier is titanium dioxide. Titanium dioxide provides the optimum effect in a composition which is stored within another because it presents a silvery interface between the first and second compositions. This is an attractive consumer positive.

The level of opacifier present, namely, is from 0.25 to 2%, preferably 0.3 to 1.2%, more preferably from 0.4 to 0.7% and most preferably 0.5% by weight of the second composition. This provides the maximum level of opacification without damaging the rheology profile of this type of composition so much that no amount of balancing can repair.

The toothpaste also contains an inorganic or a natural or synthetic thickener or gelling agent in proportions of about 0.10 to about 15% by weight depending on the material chosen. Natural thickeners will be present at much lower levels than inorganic thickeners. Natural thickeners will be present at from 0.2 to about 1% by weight of the phase in which they are present while inorganic thickeners may be present at from 2 to 15% by weight, preferably from 8 to 12% by weight of the phase in which they are present. These proportions of thickeners in the dentifrice compositions of the present invention form an extrudable, shape-retaining product which can be squeezed from a tube onto a toothbrush and will not fall between the bristles of the brush but rather, *will* substantially maintain its shape thereon. Suitable thickeners or gelling agents useful in the practice of the present invention include inorganic thickening silicas such as amorphous silicas available from Huber Corporation under the trade designation Zeodent 165, Irish moss, iota-carrageenan, gum tragacanth, and polyvinylpyrrolidone.

Preferably the thickening silica in the second composition is present at from 5 to 15% by weight of the second composition, preferably from 7 to 13% by weight and most preferably from 8 to 10% by weight.

The amount of thickener in the first composition differs from the amount of thickener in the second composition in that there is more in the first composition than in the second composition. This provides the balancing between the otherwise different rheology profiles between the two compositions caused by the opacifier in the second composition.

The first composition comprises from 0.2 to 2.0y% by weight of the first composition more thickener than is present in the second composition as a weight percentage of the second composition. Preferably, this thickener is thickening silica. Preferably, the first composition comprises 0.3 to 1.0y%, more preferably 0.4 to 0.8y% and most preferably 0.45 to 0.55% by weight more thickener than is present in the second composition as a weight present of the second composition.

In the most preferred embodiment, the weight percent amount of thickening silica in the first composition is the same as the weight percent amount in the second composition but in addition has half the amount of opacifier in the second composition added as thickening silica in the first composition.

In a preferred embodiment the second composition constitutes from 5 to 25% by volume of the total toothpaste. is Preferably, the inner second composition constitutes from 11 to 20% and more preferably from 13 to 18% by volume of the toothpaste.

In a preferred embodiment the first composition is visually-clear. By this is meant that the inner, second composition can be seen through the outer, first composition.

The first and second compositions may be the same or different with regard to their principle components, i.e. thickeners, actives, structurants and abrasives. Where the first and second phases are essentially the same they may differ in minor components such as colours or flavours. Preferably, the compositions are substantially identical other than with regard to minor components such as colours or flavours.

In a preferred embodiment at least one of the phases is coloured. Preferably, the outer, first composition is coloured. Preferred colours include green, red, orange, yellow, blue, gold and purple.

In a further preferred embodiment the compositions comprise abrasive silica. The preferred abrasive silicas used in the present invention is a silica with a low refractive index. It may be used as the sole abrasive silica, or in conjunction with a low level of other abrasive silicas, e.g. those according to EP 236070. The low refractive index silicas, used as abrasives in the present invention are preferably silicas with an apparent refractive index (R.I.) in the range of 1.41- 1.47, preferably 1.435- 1.445, preferably having a weight mean particle size of between 5 and 15 mm, a BET (nitrogen) surface area of between 10 and 100 m²/g and an oil absorption of about 70- 150 cm³/100 g, but abrasive silicas with a lower apparent refractive index may also be used. Typical examples of suitable low refractive index abrasive silicas (e.g. having an R.I. of between 1.435 and 1.445) are Tixosil 63 and 73 ex Rhone Poulenc; Sident 10 ex Degussa; Zeodent 113 ex Zeofinn; Zeodent 124 ex Huber, Sorbosil AC 77 ex Crosfield Chemicals (having an R.I. of approximately 1.440). The amount of these silicas in the composition generally ranges from 5-60% by weight, usually 5-20% by weight.

The compositions of the toothpaste according to the invention are manufactured using standard processes. They may be extruded into a container for dispensing by equipment such as that discussed in detail in WO 99/01342, i.e. a coaxial nozzle assembly attached to standard equipment.

In a most preferred embodiment the compositions according to the toothpaste of the invention have viscosities as measured on a Brookfield RV DV-1 viscometer fitted with a Helipath stand at 25°C and 5rpm using a spindle are from 150 000 Pa.s and 250 000 mPa.s. Such viscosities provide the best performance with regard to extrusion into the container and also from the container by the consumer. The phases within this viscosity range are much more stable, physically during extrusion, that other phases.

The toothpaste composition according to the present invention can comprise an agent selected from the group consisting of anti-carries agents, tooth whitening agents, anti-tartar agents, anti-malodour agents, anti-gingivitis agents and mixtures thereof.

The toothpaste composition will comprise further ingredients which are common in the art, such as:
antimicrobial agents, e.g. chlorhexidine, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2, 2' methylenebis-(4-chloro-6-bromophenol);
anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
colouring agents;
pH-adjusting agents;
sweetening agents;
pharmaceutically acceptable carriers, e.g. starch, sucrose, water or water/alcohol systems etc.;
surfactants, such as anionic, nonionic, cationic and zwitterionic or amphoteric surfactants;
particulate abrasive materials such as silicas, aluminas, calcium carbonates, dicalciumphosphates, calcium pyrophosphates, hydroxyapatites, trimetaphosphates, insoluble hexametaphosphates and so on, including agglomerated particulate abrasive materials, usually in amounts between 3 and 60% by weight of the oral care composition. Preferred abrasives are chalk and silica, more preferably fine ground natural chalk.

Humectants such as glycerol, sorbitol, propyleneglycol, xylitol, lactitol etc.;
binders and thickeners such as sodium carboxymethyl-cellulose, hydroxyethyl cellulose (Natrosol^{®}), xanthan gum, gum arabic etc. as well as synthetic polymers such as polyacrylates and carboxyvinyl polymers such as Carbopol^{®};
polymeric compounds which can enhance the delivery of active ingredients such as antimicrobial agents can also be included;
buffers and salts to buffer the pH and ionic strength of the oral care composition; and
other optional ingredients that may be included are e.g. bleaching agents such as peroxy compounds e.g. potassium peroxydiphosphate, effervescing systems such as sodium bicarbonate/citric acid systems, colour change systems, and so on.

Liposomes may also be used to improve delivery or stability of active ingredients.

In a second aspect the present invention provides a toothpaste container comprising a first and second phase as described in the first aspect of the invention stored within a tubular container, the container comprising a tubular body which is crimped at one end and comprises a dispensing assembly at the other through which the composition is extruded by the consumer.

Preferably, the tubular container is transparent or translucent so that the inner phase can be seen within the outer phase within the container.

An embodiment of the invention is now discussed in the nonlimiting example.

### EXAMPLE 1

This composition comprises a first composition and a second composition. The second composition is visually clear and the inner phase is opaque. The composition comprises 85% v/v of the first phase and 15% v/v of the second phase.

| **Ingredient** | **% (w/w) of the first phase** | **% (w/w) of the second phase** |
|---|---|---|
| water | 12,73 | 12,48 |
| sorbitol (70%aq) | 63,00 | 63,00 |
| sodium fluoride | 0,32 | 0,32 |
| polyethylene glycol (PEG 32) | 4,00 | 4,00 |
| titanium dioxide | 0,00 | 0,50 |
| thickening silica | 9,25 | 9,00 |
| Abrasive silica | 8,00 | 8,00 |
| sodium carboxymethylcellulose | 0,90 | 0,90 |
| sodium lauryl sulphate | 1,80 | 1,80 |
| flavours and colours | trace | trace |

### EXAMPLE 2

The following experiment demonstrates the change in rheology associated with the addition of a small amount of opacifier, in this case titanium dioxide.

The graphs shows the change in viscosity during a change in shear rate. The change in shear rate represents the different shears experienced during extrusion into the tube and also extrusion from the tube.

The viscosity of the formulation is reduced with the addition of titanium dioxide. Since only one of the formulations has titanium dioxide, this reduction means that the two formulations represents the two distinct phases of the toothpaste. Accordingly, post addition of titanium dioxide the formulations are not rheology matched.

As can be seen in Graph 1 the viscosity of the formulation is reduced by adding 0.5% by weight of the phase titanium dioxide. From Graph 2 can be seen that the viscosity can be raised by adding 0.25% thickening silica in place of 0.5% titanium dioxide.

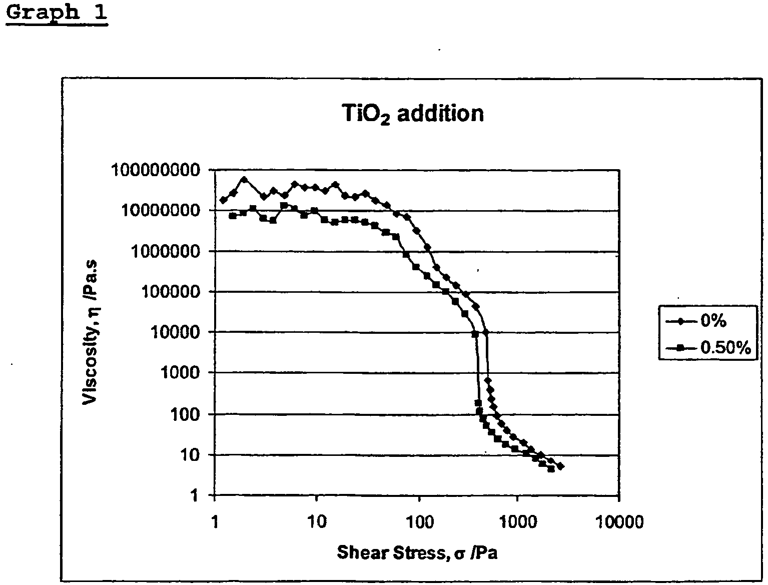

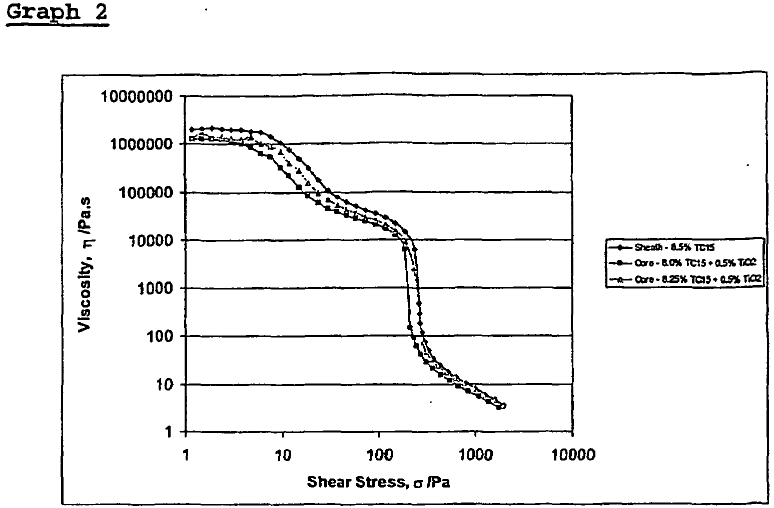

## Claims

1. A toothpaste which comprises first and second compositions, the first and second compositions comprising water, humectant and thickener and the second composition disposed coaxially within the first composition, the first composition being a gel and the second composition being opaque, the second composition comprising an opacifier at y% by weight of the second composition and the first composition being substantially devoid of any such opacifier, and the first composition comprising 0.2 to 2.0y% by weight of the first composition more thickener than the amount of thickener which is present in the second composition as a weight percent of the second composition, wherein the opacifier is titanium dioxide, and wherein y is from 0.25 to 2.

2. A toothpaste according to claim 1 wherein y is from 0.3 to 1.2.

3. A toothpaste according to claim 2 wherein y is from 0.4 to 0.7.

4. A toothpaste according to claim 3 wherein y is 0.5.

5. A toothpaste according to any preceding claim wherein the first composition comprises from 0.3 to 1y% by weight of the first composition more thickener than is present in the second composition.

6. A toothpaste according to any preceding claim wherein the first composition comprises from 0.4 to 0.8y% by weight of the first composition more thickener than is present in the second composition.

7. A toothpaste according to any preceding claim wherein the first composition comprises from 0.45 to 0.55y% by weight of the first composition more thickener than is present in the second composition.

8. A toothpaste according to any preceding claim wherein the first composition comprises from 0.5y% by weight of the first composition more thickener than is present in the second composition.

9. A toothpaste according to any preceding claim wherein the thickener is thickening silica.

10. A toothpaste according to any preceding claim wherein the first composition is a visually-clear gel.

11. A toothpaste according to any preceding claim wherein the first and second compositions differ only in the level of thickener, and in the selection of colours and flavours.

12. A toothpaste according to any preceding claim wherein the second composition constitutes from 5 to 25% by volume of the toothpaste.

13. A toothpaste container comprising a first and second composition as claimed in any preceding claim the second composition being disposed coaxially within the first composition, the container comprising a tubular body which is crimped at one end and comprises a dispensing assembly at the other through which the composition is extruded by the consumer.

## Patentansprüche

1. Zahnpasta, die eine erste und eine zweite Zusammensetzung umfasst, wobei die erste und die zweite Zusammensetzung Wasser, Feuchthaltemittel und Verdickungsmittel umfassen, und die zweite Zusammensetzung koaxial in der ersten Zusammensetzung angeordnet ist, wobei die erste Zusammensetzung ein Gel ist und die zweite Zusammensetzung opak ist, die zweite Zusammensetzung ein Trübungsmittel mit y Gew.-% der zweiten Zusammensetzung umfasst und die erste Zusammensetzung im Wesentlichen frei von einem solchen Trübungsmittel ist, und wobei die erste Zusammensetzung 0,2 bis 2,0 y Gew.-% der ersten Zusammensetzung mehr Verdickungsmittel als die Menge des Verdickungsmittels, die in der zweiten Zusammensetzung, als Gewichtsprozent der zweiten Zusammensetzung vorliegt, umfasst, wobei das Trübungsmittel Titandioxid ist und wobei y von 0,25 bis 2 ist.

2. Zahnpasta gemäß Anspruch 1, wobei y von 0,3 bis 1,2 ist.

3. Zahnpasta gemäß Anspruch 2, wobei y von 0,4 bis 0,7 ist.

4. Zahnpasta gemäß Anspruch 3, wobei y 0,5 ist.

5. Zahnpasta gemäß einem vorangehenden Anspruch, wobei die erste Zusammensetzung von 0,3 bis 1 y Gew.-% der ersten Zusammensetzung mehr Verdickungsmittel umfasst als in der zweiten Zusammensetzung vorliegt.

6. Zahnpasta gemäß einem vorangehenden Anspruch, wobei die erste Zusammensetzung von 0,4 bis 0,8 y Gew.-% der ersten Zusammensetzung mehr Verdickungsmittel umfasst als in der zweiten Zusammensetzung vorliegt.

7. Zahnpasta gemäß einem vorangehenden Anspruch, wobei die erste Zusammensetzung von 0,45 bis 0,55 y Gew.-% der ersten Zusammensetzung mehr Verdickungsmittel umfasst als in der zweiten Zusammensetzung vorliegt.

8. Zahnpasta gemäß einem vorangehenden Anspruch, wobei die erste Zusammensetzung ab 0,5 y Gew.-% der ersten Zusammensetzung mehr Verdickungsmittel umfasst als in der zweiten Zusammensetzung vorliegt.

9. Zahnpasta gemäß einem vorangehenden Anspruch, wobei das Verdickungsmittel verdickendes Siliciumdioxid ist.

10. Zahnpasta gemäß einem vorangehenden Anspruch, wobei die erste Zusammensetzung ein visuell klares Gel ist.

11. Zahnpasta gemäß einem vorangehenden Anspruch, wobei sich die erste und zweite Zusammensetzung nur in dem Gehalt an Verdickungsmittel und in der Auswahl von Farben und Aromastoffen unterscheiden.

12. Zahnpasta gemäß einem vorangehenden Anspruch, wobei die zweite Zusammensetzung 5 bis 25 Vol.-% der Zahnpasta bildet.

13. Zahnpastabehälter, der eine erste und zweite Zusammensetzung, wie in einem vorangehenden Anspruch beansprucht, umfasst, wobei die zweite Zusammensetzung koaxial in der ersten Zusammensetzung angeordnet ist, der Behälter einen röhrenförmigen Behälter umfasst, der an einem Ende gefaltet ist und eine Dosieranordnung an dem anderen umfasst, durch welche die Zusammensetzung vom Verbraucher extrudiert wird.

## Revendications

1. Pâte dentifrice qui comprend des première et seconde compositions, les première et seconde compositions comprenant de l'eau, un humectant et un épaississant et la seconde composition étant disposée coaxialement à l'intérieur de la première, la première composition étant un gel et la seconde composition étant opaque, la seconde composition comprenant un opacifiant à y % en poids de la seconde composition et la première composition étant essentiellement dépourvue de tout opacifiant de ce type, et la première composition comprenant de 0,2 à 2,0y % en poids de la première composition d'épaississant en plus que la quantité d'épaississant qui est présente dans la seconde composition en pourcentage en poids de la seconde composition, dans laquelle l'opacifiant est le dioxyde de titane, et dans laquelle y est de 0,25 à 2.

2. Pâte dentifrice selon la revendication 1 dans laquelle y vaut de 0,3 à 1,2.

3. Pâte dentifrice selon la revendication 2 dans laquelle y vaut de 0,4 à 0,7.

4. Pâte dentifrice selon la revendication 3 dans laquelle y est 0,5.

5. Pâte dentifrice selon l'une quelconque des revendications précédentes dans laquelle la première composition comprend de 0,3 à 1y % en poids de la première composition d'épaississant en plus que la quantité présente dans la seconde composition.

6. Pâte dentifrice selon l'une quelconque des revendications précédentes dans laquelle la première composition comprend de 0,4 à 0,8y % en poids de la première composition d'épaississant en plus que la quantité présente dans la seconde composition.

7. Pâte dentifrice selon l'une quelconque des revendications précédentes dans laquelle la première composition comprend de 0,45 à 0,55y % en poids de la première composition d'épaississant en plus que la quantité présente dans la seconde composition.

8. Pâte dentifrice selon l'une quelconque des revendications précédentes dans laquelle la première composition comprend 0,5y % en poids de la première composition d'épaississant en plus que la quantité présente dans la seconde composition.

9. Pâte dentifrice selon l'une quelconque des revendications précédentes dans laquelle l'épaississant est une silice épaississante.

10. Pâte dentifrice selon l'une quelconque des revendications précédentes dans laquelle la première composition est un gel transparent à l'oeil nu.

11. Pâte dentifrice selon l'une quelconque des revendications précédentes dans laquelle les première et seconde compositions ne diffèrent que par le niveau d'épaississant, et par le choix des couleurs et des saveurs.

12. Pâte dentifrice selon l'une quelconque des revendications précédentes dans laquelle la seconde composition représente de 5 à 25 % en volume de la pâte dentifrice.

13. Contenant pour pâte dentifrice comprenant une première et une seconde composition selon l'une quelconque des revendications précédentes, la seconde composition étant disposée coaxialement à l'intérieur de la première, le contenant comprenant un corps tubulaire qui est serti à une extrémité et comprend un ensemble distributeur à l'autre par lequel la composition est extrudée par le consommateur.
